**Europäisches Patentamt**

**European Patent Office**

**Office européen des brevets**

(11) Veröffentlichungsnummer: **0 129 188**
A2

# (12) EUROPÄISCHE PATENTANMELDUNG

(21) Anmeldenummer: **84106694.7**

(22) Anmeldetag: **12.06.84**

(51) Int. Cl.$^3$: **C 04 B 35/48**

(30) Priorität: **16.06.83 DE 3321857**
**16.12.83 DE 3345659**

(43) Veröffentlichungstag der Anmeldung:
**27.12.84 Patentblatt 84/52**

(84) Benannte Vertragsstaaten:
**AT BE CH DE FR GB IT LI LU NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Claussen, Nils, Dr.**
**Forschenweg 24**
**D-7250 Leonberg(DE)**

(72) Erfinder: **Rühle, Manfred, Dr.**
**Aspergstrasse 21**
**D-7257 Ditzingen(DE)**

(72) Erfinder: **Petzow, Günter, Prof. Dr.**
**Tannenweg 7**
**D-7022 Leinfelden-Echterdingen 1(DE)**

(74) Vertreter: **Weickmann, Heinrich, Dipl.-Ing. et al,**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86(DE)**

(54) **Keramikkörper aus Zirkoniumdioxid (ZrO2) und Verfahren zu seiner Herstellung.**

(57) Ein keramischer Körper aus gegebenenfalls $Al_2O_3$ enthaltendem, mit Ytriumoxid und/oder einem oder mehreren Seltenerdoxiden (z.B. $CeO_2$) und/oder MgO und/oder CaO teilstabilisierten Zirkoniumdioxid ist mit 0,5 bis 5 Mol-% $Y_2O_3$ und/oder 5 bis 12 Mol-% MgO und/oder CaO und/oder $CeO_2$ oder einem oder mehreren Seltenerdoxiden teilstabilisiert liegt zu 30 bis 100% in der tetragonalen Gittermodifikation vor und weist im Oberflächenbereich einen gegenüber dem Durchschnittsgehalt um 2 bis 20 Mol- höheren Gehalt an $Y_2O_3$, $CeO_2$, MgO, CaO bzw. Seltenerdoxid auf, derart, daß der Körper von einer höher stabilisierten tetragonalen oder einer überwiegend in der kubischen Gitterform vorliegenden dünnen, PSZ-artigen Schicht überzogen ist. Zur Herstellung wird die Oberfläche eines bereits gesinterten oder nur vorverfestigten Formlings aus teilstabilisiertem $ZrO_2$ in innigen Kontakt mit $Y_2O_3$, $CeO_2$, MgO, CaO oder/und einem anderen Seltenerdoxidpulver oder einem mindestens 12 Mol-% $Y_2O_3$ und/oder andere Stabilisatoroxide enthaltendem $ZrO_2$ Pulver gebracht, und dann bei 1000 bis 1600°C so lange geglüht, bis sich eine höher stablisierte tetragonale bzw. überwiegend kubische Oberflächenschicht von 0,1 bis 200 $\mu$m Dicke und 2 bis 20 Mol-% höherem Gehalt an $Y_2O_3$, $CeO_2$, MgO, CaO bzw. Seltenerdoxid gebildet hat.

B e s c h r e i b u n g

Feinkörnige, mit $Y_2O_3$, $CeO_2$ und/oder anderen Seltenerdoxiden bzw. auch grobkörnige mit MgO oder CaO teilstabilisierte $ZrO_2$-Körper gehören zu den polykristallinen
Keramiken, die die höchsten bisher gemessenen Festigkeiten und Bruchwiderstandswerte aufweisen. Die Hauptursache dafür liegt in der spannungsinduzierten Umwandlung
der tetragonalen Gittermodifikation in die monokline
Raumtemperaturmodifikation. Zum Beispiel werden
$Y_2O_3$-haltige Körper meistens mit einem $Y_2O_3$-Anteil
zwischen 1 und 6 Mol-% entweder im tetragonalen Einphasenfeld bzw. im kubisch/tetragonalen Zweiphasengebiet bei
Temperaturen zwischen 1400° und 1550°C gesintert,
heißgepreßt oder heißisostatisch gepreßt (gehippt). Ihr
Gefüge besteht danach aus einem feinkörnigen (0.1 - 1.0
µm), tetragonalen Anteil (bis zu 100 %) und etwas
gröberen (1 - 10 µm), kubischen Körnern (bei hohen
$Y_2O_3$-Anteilen: 3.5 - 6.0 Mol-%). Zur Erhöhung der Härte
und des E-Moduls können die Körper $Al_2O_3$ in größeren
Mengen enthalten.

MgO- bzw. CaO-haltige $ZrO_2$-Körper werden meistens bei
Temperaturen zwischen 1690 und 1800°C im kubischen Einphasengebiet gesintert; sie sind daher grobkörniger (50
bis 70 µm).

Der entscheidende Nachteil dieser superfesten, besonders der $Y_2O_3$-haltigen, Keramikkörper liegt darin, daß
sie nach längerer Glühdauer bei Temperaturen zwischen
200° und 550°C an Luft ihre Festigkeit drastisch
verlieren; diese Festigkeitsabnahme wird mit zunehmender Luftfeuchtigkeit bzw. höherem Wasserdampfdruck

0129188

stark beschleunigt (O. T. Masaki, K. Kobayashi, Proc.
Ann. Meeting Jap.Ceram.Soc. 1981). Sogar in warmen,
wässrigen Lösungen kann es zu einer Zersetzung der
Körper kommen. Die Ursache dafür ist noch nicht geklärt.
Es wird jedoch angenommen, daß durch Spannungsrißkorrosion an den Korngrenzen die mechanischen Verspannungen
der tetragonalen Körper weggenommen werden und so eine
Umwandlung in die monokline Form eintritt, oder daß
durch andere diffusionsgesteuerte Mechanismen
Martensitkeime an der Oberfläche gebildet werden und
damit die Umwandlung auslösen, was schließlich zur
Zerstörung der Körper führt.

Dieser entscheidende Nachteil macht die neue Klasse der
sogenannten TZP-Keramiken (TZP: Tetragonal Zirconia
Polycrystals eine Literaturübersicht über TZP-Keramiken
ist in dem Buch "Science and Technology of Zirconia
II", Advances in Ceramics Vol II, 1984, enthalten) an
Luft nur für Anwendungstemperaturen bis ca. 200°C
geeignet, obwohl solche Keramiken besonders beim
Einsatz in Wärmekraftmaschinen wesentliche Vorteile
bringen würden. Auch für die Anwendung als Biokeramik
(Hüftprothese) wäre dieses Phänomen von Nachteil.

Einen ähnlichen Nachteil weisen auch konventionelle
MgO-teilstabilisierte $ZrO_2$-Keramiken (Mg - PSZ) bei
Langzeitglühungen bei etwas höheren Temperaturen auf
(700 bis 1000°C). Aufgrund der schnellen Diffusion bzw.
Abdampfrate des MgO tritt hier, besonders bei leicht
reduzierender Atmosphäre, eine Oberflächenzersetzung
auf.

Überraschenderweise wurde nun gefunden, und hierauf beruht die Erfindung, daß in gesinterten Proben, die in einer an $Y_2O_3$-, $CeO_2$-, MgO- oder CaO-reichen Umgebung, z. B. einem Pulverbett aus $Y_2O_3$ oder MgO, geglüht wurden, dieser Zersetzungsprozeß nicht oder nur vermindert auftritt.

Der Erfindung liegt daher die Aufgabe zugrunde, die oben beschriebene Festigkeitsabnahme bzw. Oberflächenzersetzung bei keramischen Körpern aus Zirkoniumdioxid zu verringern oder zu beseitigen.

Gelöst wird diese Aufgabe durch einen keramischen Körper aus gegebenenfalls $Al_2O_3$ enthaltendem, mit Yttriumoxid und/oder $CeO_2$ und/oder einem oder mehreren Seltenerdoxiden und/oder MgO teilstabilisierten Zirkoniumdioxid, welcher dadurch gekennzeichnet ist, daß er mit 0,5 bis 5 Mol-% $Y_2O_3$ und/oder 2 bis 12 Mol% MgO und/oder CaO und einem oder mehreren Seltenerdoxiden (z. B. $CeO_2$) teilstabilisiert ist, zu 30 bis 100 % in der tetragonalen Gittermodifikation vorliegt und im Oberflächenbereich einen gegenüber dem Durchschnittsgehalt um 2 bis 20 Mol-% höheren Gehalt an $Y_2O_3$ bzw. Seltenerdoxid und/oder MgO und/oder CaO aufweist, derart daß der Körper entweder von einer überwiegend in der kubischen oder in einer höher stabilisierten tetragonalen Gitterform vorliegenden dünnen Schicht überzogen ist. Es versteht sich von selbst, daß eine teilstabilisierte kubische Schicht durch Anlaßglühen (peak aging) bei PSZ-üblichen Temperaturen (1100 bis 1420°C) in eine PSZ-artige Schicht (d. h. kubische Körner mit tetragonalen Ausscheidungen) überführt werden kann.

Als dünne Oberflächenschicht im Sinne der Erfindung
wird hierbei eine Schicht mit einer Dicke von 0,1 bis
200 µm, vorzugsweise von 0,3 bis 30 µm verstanden. Der
erfindungsgemäße Körper auf Basis Zirkoniumdioxid wird
hergestellt, indem man ihn in einer Umgebung glüht,
welche reich an $Y_2O_3$, $CeO_2$, MgO, CaO oder/und Seltenerdoxiden ist. Im folgenden wird die Erfindung anhand
der Anwendung von $Y_2O_3$, $CeO_2$, MgO, CaO beschrieben. Es
versteht sich jedoch, daß dies in gleicher Weise auch
für andere Seltenerdoxide gilt. Diese Oberflächenstabilisierung bzw. -vergütung ist ebenfalls für konventionelle MgO- bzw. CaO-teilstabilisierte $ZrO_2$-Keramiken von
Vorteil.

Für die Herstellung des erfindungsgemäßen keramischen
Körpers kann entweder von der bereits fertig gesinterten oder heißisostatisch gepreßten Keramik oder aber
von einem bei relativ niedriger Temperatur (z. B. Raumtemperatur) vorverfestigten Grünkörper ausgegangen
werden. Die Keramik oder der Grünkörper werden nun mit
einer Oberflächenschicht aus $Y_2O_3$, $CeO_2$, MgO, CaO, etc.
versehen und zwar entweder in Form einer aufgepreßten
Pulverschicht oder eines $Y_2O_3$- bzw. MgO-haltigen
Schlickers, der z. B. aufgesprüht oder in Form eines
Bades zum Tränken der Oberfläche angewendet werden
kann. Die so behandelten Körper werden anschließend bei
Temperaturen zwischen 1000 und 1600°C geglüht bzw. gesintert, wobei die Dauer der Behandlung zwischen etwa
10 Minuten und etwa 100 Stunden liegen kann. Die erwünschte Oberflächenstabilisierung wird auch besonders
vorteilhaft dadurch erreicht, daß die Keramik bzw. der
Grünkörper in einem Pulverbett aus $Y_2O_3$ und/oder $CeO_2$
und/oder MgO und/oder CaO geglüht bzw. gesintert wird.
Dabei werden solche Bedingungen bevorzugt, bei denen
die gewünschte Eindiffusion in möglichst kurzen Zeiten
und gleichzeitig eine PSZ-artige Schicht erzielt wird.

Zur Herstellung des keramischen Körpers selbst kann
dieser entweder durch Mischen der Oxide oder nach
naßchemischen Verfahren wie Solgel, Kopräzipitation,
Sprühreaktion wäßriger Lösungen oder aus feinen, im
Schmelzfluß gewonnenen, homogenen und mit $Y_2O_3$, $CeO_2$,
MgO und/oder CaO vorlegierten feinen Pulvern geformt
und dann bei Temperaturen, die im allgemeinen zwischen
1350 und 1550°C liegen, gesintert oder heißisostatisch
gepreßt (gehippt) oder gesintert und nachgehippt
werden. Die fertige Keramik wird dann, wie oben bereits
erwähnt, mit $Y_2O_3$, $CeO_2$, MgO, CaO etc. überzogen bzw.
in einem entsprechenden Pulverbett geglüht, bis die mit
$Y_2O_3$, $CeO_2$, MgO, CaO etc. angereicherte Oberflächenschicht gebildet wurde.

Beim Aufbringen einer stabilisatorreichen Schicht auf
einen Grünkörper wird der Körper üblicherweise mit
einem geringen Druck, also etwa 100 MPa, vorgeformt und
dann bei höherem Druck, beispielsweise 200 bis 650 MPa,
nachgepreßt. In den meisten Fällen aber ist die bevorzugte Ausführungsform das Sintern des fertig gepreßten
Körpers, bzw. das Glühen einer fertig gesinterten und
bearbeiteten Körpers in einem MgO-, bzw. $Y_2O_3$- und/oder
$CeO_2$-haltigen Pulverbett.

Die erfindungsgemäßen keramischen Formkörper zeigen im
Vergleich zu Proben, die unter sonst gleichen Bedingungen, jedoch ohne die beschriebene Oberflächenbehandlung
hergestellt wurden, bei einer Behandlung zur beschleunigten Alterung, die in vierstündigem Glühen bei Temperaturen zwischen 250°C und 400°C und Wasserdampfdrücken
von 4 bis 15 bar besteht, kaum eine Beeinflussung.

Röntgenographisch werden nach dieser Behandlung bei den erfindungsgemäßen keramischen Körpern nur die kubischen und tetragonalen Reflexe des nicht der beschleunigten Alterung unterworfenen Körpers erkennbar, während die Vergleichsproben starke monokline Reflexe zeigen, die ein Hinweis auf begonnene Zersetzung sind. Dabei wurden die besten Ergebnisse dann erhalten, wenn die dünne Oberflächenschicht durch Glühen der fertig gesinterten Proben in MgO-, $Y_2O_3$-, $CeO_2$ oder CaO-Pulver erzeugt wurde oder durch Behandlung mit $Y_2O_3$-Pulver oder einem mit mindestens 12 Mol-% $Y_2O_3$ enthaltenden $ZrO_2$-Pulver erfolgte, wobei die Oberflächenschicht auf den mit niedrigem $Y_2O_3$-Zusatz stabilisierten (0,5 bis 5, vorzugsweise 2 bis 4 Mol-% $Y_2O_3$) $ZrO_2$-Preßkörper aufgepreßt oder als wässrige Pulversuspension aufgebracht und gesintert wurde. Aber wie auch immer die Herstellung der Oberflächenschicht erfolgt, ist eine wichtige Voraussetzung für die Erzielung der schützenden Wirkung des Stabilisator-haltigen Überzugs ein möglichst enger Kontakt mit der zu erhitzenden bzw. zu sinternden $ZrO_2$-Probenoberfläche.

Die dünne, im allgemeinen 0,5 bis 30 µm tiefe Stabilisator-reiche $ZrO_2$-Oberflächenschicht, die durch die erfindungsgemäße Behandlung entsteht, scheint einen Schutz gegen den thermischen Langzeitzerfall darzustellen. Diese Schicht kann zur Kornfeinung auch $Al_2O_3$ enthalten. Einen ähnlich positiven Effekt zeigen, wie oben schon erwähnt, vermutlich auch andere Seltenerdoxide. Aufgrund der äußerst langsamen Diffusion von $Y_2O_3$ in $ZrO_2$ bei Temperaturen unterhalb 1000°C stellt diese Schicht vor allem einen thermisch stabilen Schutz für TZP-Keramiken, aber auch für mit Mg- bzw. Ca-teilstabilisiertes, konventionelles $ZrO_2$ (Mg-, Ca-PSZ) dar.

Die folgenden Beispiele erläutern die Erfindung weiter.


B e i s p i e l    1

Proben wurden aus reaktionsgesprühten (EDS-Pulver: Evaporation Decomposition of Solutions, Am. Ceram. Soc. Bull. $\underline{50}$ (1977) 1023) Pulvern, die 2 Mol-% $Y_2O_3$ und 1,5 Vol.-% $Al_2O_3$ enthielten und 4 Stunden in einem Attritor mit $SiO_2$-haltigen $Al_2O_3$-Mahlkugeln in Wasser gemahlen und sprühgetrocknet waren, bei 630 MPa isostatisch gepreßt und 2 Stunden bei 1450 °C an Luft gesintert. Die Röntgenreflexe zeigten danach ein überwiegend tetragonales Gefüge an (Korngröße ca. 0,4 µm). Daraus getrennte Biegeproben wiesen im überschliffenen Oberflächenzustand eine Festigkeit von 920 MPa (Typ I) und nach einer 36 h Glühung bei 1350 °C in einem $Y_2O_3$-Pulverbett eine Festigkeit von 810 MPa (Typ II) auf. Nach einer Glühung aller Proben in Autoklav bei 400°C für 4 Stunden bei 4 bar Wasserdampfdruck betrug die Festigkeit des Typs I nur noch 420 MPa, während die des Typs II 740 MPa aufwies.


B e i s p i e l    2

Proben aus einem Pulver, das wie in Beispiel 1 hergestellt und behandelt wurde, jedoch nur 2 Mol-% $Y_2O_3$ enthielt, wurde wie in Beispiel 1 geformt. Auf den zylindrischen Preßkörper wurde eine wäßrige $Y_2O_3$-Pulversuspension, die zum Teil in die Oberflächenporen eindrang, aufgebracht; anschließend wurden die beschichteten Preßkörper (Typ I) bei 1500°C 2 Stunden gesintert und danach zusammen mit identischen Proben ohne Beschichtung (Typ II) der Autoklavenbehandlung nach Beispiel 1
unterzogen. Nach dieser Behandlung zeigte der Typ I nur
tetragonale und kubische, der Typ II dagegen tetragonale
und große monokline Röntgenreflexe, was auf die thermische Zersetzung der unbeschichteten Proben hinweist.

B e i s p i e l     3

Proben aus dem Pulver aus Beispiel 1 wurden bei einem
Druck von 100 MPa isostatisch gepreßt, anschließend mit
einer Suspension aus 12 Mol-% $Y_2O_3$-haltigen $ZrO_2$-Pulver
besprüht (Schichtdicke ca. 40 bis 200 μm), danach bei
630 MPa isostatisch nachgepreßt und wie in Beispiel 1
gesintert. Nach der Glühbehandlung im Autoklav (wie
Beispiel 1) war keine thermische Zersetzung der Oberfläche festzustellen.

B e i s p i e l     4

Proben nach Beispiel 2 wurden mit derselben Suspension,
nur diesmal mit 20 Vol.-% $Al_2O_3$-Zusatz, beschichtet und
sonst wie in Beispiel 1 behandelt. Auch hier konnte
nach der Glühbehandlung im Autoklav keine Zersetzung
festgestellt werden.

B e i s p i e l     5

Dem Pulver aus Beispiel 1 wurde 50 Vol.-% $Al_2O_3$-Pulver
(Pechinée Ugine Kuhlman, A6) und wie in Beispiel 1
attritiert. Isostatisch gepreßte Zylinder (ca. 1x1 cm Ø)
wurden bei 1500°C zum Teil ohne (Typ I) und zum Teil

mit (Typ II) einem Schlickerüberzug aus 50 Gew.-% $Y_2O_3$ und 50 Gew.-% $CeO_2$ gesintert. Danach enthielt der Typ I auf der geschliffenen Oberfläche neben $Al_2O_3$ ausschließlich tetragonales $ZrO_2$ (röntgenographisch gemessen), während Typ II zusätzlich kubische Anteile zeigte. Nach einer Autoklavbehandlung wie in Beispiel 1, nur mit 8 bar Wasserdampfdruck, enthielt die Oberfläche des Typs I einen hohen Anteil an monoklinem $ZrO_2$, während der Typ II keine meßbare Veränderung erkennen ließ.


B e i s p i e l    6


Ein kopräzipiertes, 2,2 Mol% $Y_2O_3$-haltiges $ZrO_2$-Pulver wurde isostatisch bei 620 MPa gepreßt; die Proben wurden anschließend bei 1500°C, 2 Stunden an Luft gesintert. Die so gefertigten Körper enthielten ausschließlich tetragonale Körner mit einer mittleren Größe von 0,4 µm (Materialtyp A). Ein ähnlich hergestelltes kommerzielles Material mit 3 Mol% $Y_2O_3$ enthielt ca. 80 % tetragonale Körner ( 0,4 µm) und ca. 20% kubische Körner ( 5 µm) (Materialtyp B).

Die Materialtypen A und B wurden einem Autoklavtest mit einem Wasserdampfdruck von 5 bar bei 250°C über 2 Stunden ausgesetzt, wobei beide Typen sich stark zersetzten, d. h. überwiegend monokline Reflexe an der Oberfläche aufwiesen; Typ A war sogar zum Teil völlig zerfallen.

Typ A und B wurden nun jeweils 2 Stunden in einem Pulverbett aus jeweils $Y_2O_3$, $CeO_2$, $TiO_2$, MgO und Cao bei verschiedenen Temperaturen geglüht. Die Glühtemperaturen sowie die Ergebnisse des anschließenden Autoklavtests sind in Tabelle 1 aufgeführt. Daraus geht

hervor, daß außer $TiO_2$ alle übrigen Oxide sich positiv, besonders bei höheren Temperaturen auswirken. Eine MgO-Pulverbettglühung ist auch bei relativ geringen Temperaturen (1120°C) schon wirksam.

Typ A und B wurden als ungesinterte Preßkörper 2 Stunden bei 1500°C in einem Pulverbett aus jeweils $Y_2O_3$, $CeO_2$, CaO und MgO gesintert (an Luft). Der oben angeführte Autoklavtest führte ebenfalls zu keiner Oberflächenzersetzung.

### T a b e l l e    1

Glühbehandlung im Pulverbett, danach
Autoklavtest: 2 Stunden, 5 bar Wasserdampf, 250°C.
- starke Zersetzung
O leichte Zersetzung
+ kaum Beeinflussung der vergüteten Oberfläche

| Pulverbett | $Y_2O_3$ | | $CeO_2$ | | $TiO_2$ | | MgO | | CaO | | Material-typ |
|---|---|---|---|---|---|---|---|---|---|---|---|
| Glühtemperatur (°C) | A | B | A | B | A | B | A | B | A | B | |
| 1120 | − | − | − | − | − | − | − | − | − | O | |
| 1220 | − | O | O | O | − | − | − | O | + | + | |
| 1320 | O | + | O | + | − | − | + | + | + | + | |
| 1420 | + | + | + | + | − | O | + | + | + | + | |

B e i s p i e l   7

Ein konventionelles MgO-teilstabilisiertes $ZrO_2$
(Mg-PSZ) mit 3,3 Gew.-% MgO wurde nach einer Lösungsglühbehandlung (solution anneal) an Luft bei 1700°C für
2 Stunden und anschließendem schnellen Abkühlen auf
Raumtemperatur einer zweistündigen Glühung bei 1420°C
in $Y_2O_3$-Pulver ausgesetzt. Während der monokline Anteil
an der Oberfläche bei der unbehandelten (as-recieved)
Probe nach 100 Stunden in leicht reduzierender Atmosphäre bei 920°C von ursprünglich 15 % auf 32 % anstieg,
blieb der monokline Anteil bei der in $Y_2O_3$ geglühten
Probe unterhalb des meßbaren Bereiches, d. h. <~4 %.

Patentansprüche

1. Keramischer Körper aus gegebenenfalls $Al_2O_3$ enthaltendem, mit Yttriumoxid und/oder einem oder mehreren Seltenerdoxiden (z. B. $CeO_2$) und/oder MgO und/oder CaO teilstabilisierten Zirkoniumdioxid, d a d u r c h   g e k e n n z e i c h n e t , daß er mit 0,5 bis 5 Mol-% $Y_2O_3$ und/oder 5 bis 12 Mol-% MgO und/oder CaO und/oder $CeO_2$ oder einem oder mehreren Seltenerdoxiden teilstabilisiert ist, zu 30 bis 100 % in der tetragonalen Gittermodifikation vorliegt und im Oberflächenbereich einen gegenüber dem Durchschnittsgehalt um 2 bis 20 Mol-% höheren Gehalt an $Y_2O_3$, $CeO_2$, MgO, CaO bzw. Seltenerdoxid aufweist, derart, daß der Körper von einer höher stabilisierten tetragonalen oder einer überwiegend in der kubischen Gitterform vorliegenden dünnen Schicht überzogen ist.

2. Verfahren zur Herstellung des keramischen Körpers von Anspruch 1,   d a d u r c h   g e k e n n z e i c h n e t ,   daß die Oberfläche eines bereits gesinterten oder nur vorverfestigten Formlings aus teilstabilisiertem $ZrO_2$ in innigen Kontakt mit $Y_2O_3$, $CeO_2$, MgO, CaO oder/und einem anderen Seltenerdoxidpulver oder einem mindestens 12 Mol-% $Y_2O_3$ und/oder andere Stabilisatoroxide enthaltendem $ZrO_2$ Pulver gebracht, und dann bei 1000 - 1600°C so lange geglüht wird, bis sich eine höher stabilisierte tetragonale bzw. überwiegend kubische Oberflächenschicht von 0,1 bis 200 μm Dicke und 2 bis 20 Mol-% höherem Gehalt an $Y_2O_3$, $CeO_2$, MgO, CaO bzw. Seltenerdoxid gebildet hat.

3.  Verfahren nach Anspruch 2, d a d u r c h
    g e k e n n z e i c h n e t , daß der
    Oberflächenkontakt mit $Y_2O_3$, $CeO_2$, MgO, CaO bzw.
    Seltenerdoxid durch Einbringen in ein Pulverbett,
    Aufsprühen oder Aufpressen einer Pulverschicht
    oder Behandlung mit einer Suspension des Pulvers
    erfolgt.

4.  Verfahren nach Anspruch 2 oder 3,
    d a d u r c h    g e k e n n z e i c h n e t ,
    daß ein bei 1350 bis 1550°C gesinterter oder/und
    heißisostatisch gepreßter Körper aus
    teilstabilisiertem $ZrO_2$ behandelt wird.

5.  Verfahren nach Anspruch 2 oder 3,
    d a d u r c h    g e k e n n z e i c h n e t ,
    daß ein nicht gesinterter Preßling (Grünkörper)
    aus teilstabilisiertem $ZrO_2$ behandelt und bei 1350
    bis 1550°C gesintert wird

6.  Verfahren nach Anspruch 2 oder 3,
    d a d u r c h       g e k e n n z e i c h n e t ,
    daß ein mit 7 bis 11 Mol-% MgO teilstabilisierter
    $ZrO_2$-Körper, der bei Temperaturen zwischen 1690
    und 1800°C gesintert wurde, bei Temperaturen
    zwischen 1350 und 1550°C in $Y_2O_3$- und/oder $CeO_2$-
    und/oder anderen Seltenerdoxid-Pulvern 1 bis 5
    Stunden geglüht wird.

7.  Verfahren nach einem der Ansprüche 2 bis 6,
    d a d u r c h    g e k e n n z e i c h n e t ,
    daß ein Sinterkörper oder Grünkörper behandelt
    wird, der 2 bis 4 Mol-% $Y_2O_3$ oder Seltenerdoxid
    als Stabilisator enthält.

8. Verwendung eines keramischen Körpers gemäß
   Anspruch 1 als Bauteil in Wärmekraftmaschinen.

9. Verwendung eines keramischen Körpers gemäß
   Anspruch 1 als Knochenersatzwerkstoff.